# EUROPEAN PATENT APPLICATION

(11) **EP 4 686 452 A1**
(43) Date of publication of application: **04.02.2026**
(21) Application number: 25193011.1
(22) Date of filing: 31.07.2025
(51) Int. Cl.: A61B 17/32, A61B 17/00

(54) **MULTI-POSITIONAL GRIP FOR A SURGICAL TOOL**

(30) Priority: 31.07.2024 US 202463677816 P; 30.07.2025 US 202519284890
(71) Applicant: Arthrex, Inc, Naples, FL 34108 (US)
(72) Inventor: Caldwell, Jeremiah D., Naples, Florida, 34108 (US); Slater, Jefferey, Naples, Florida, 34108 (US); Buckman, Joshua, Naples, Florida, 34108 (US); Vayeda, Ankur, Naples, Florida, 34108 (US); Fedor, Nicholas, Naples, Florida, 34108 (US)
(74) Representative: Lohr, Jöstingmeier & Partner Patent- und Rechtsanwälte mbB

(57) **Abstract**

A multi-positional grip (12) for a surgical tool (10) includes a grip housing (14A, 14C, 14D) extending along an axis (A) between a first end (16) and a second end (18). A bottom surface (20) at least partially defines a first grasping depression (22A, 22C, 22D) and a second grasping depression (24A, 24C, 24D) spaced from the first grasping depression (22A, 22C, 22D). A top surface (26) is spaced from the bottom surface (20) by a pair of side surfaces (28). The top surface (26) includes a user interface (30) located proximate to the first end (16) that includes at least one operational input. The grip housing (14A, 14C, 14D) is configured to provide a plurality of holding positions.

## Description

### BACKGROUND

The present disclosure generally relates to a grip for a surgical tool and, more particularly, to a multi-positional grip that improves ergonomics and provides different holding positions for a caregiver's preference, during different operations, and/or at various stages of surgical procedures. Traditionally, surgical cutting tools have been implemented to improve patient outcomes by limiting tissue damage through minimally invasive techniques. However, the nature of various procedures often requires precise usage of surgical implements and tools on a patient at a variety of angles and sometimes with limited visibility. In various implementations, the disclosure may provide a multi-positional grip with gripping features that can be comfortably and securely grasped by a caregiver in different orientations.

### SUMMARY

The disclosure generally provides for a multi-positional grip for a surgical tool, the surgical tool, and a control system of the surgical tool that may be used in arthroscopic surgeries to accomplish various procedures. In various implementations, the multi-positional grip may include a grip housing with a pair of grasping depressions that permit the multi-positional grip to be steadily held by a caregiver in multiple orientations during usage. Accordingly, the disclosure may provide for improved flexibility in the usage of the surgical tool between multiple orientations based on caregiver preferences, particulars of a procedural step or stage, functionality of the surgical tool, and/or the like.

In various implementations, the disclosure may provide for a multi-positional grip for a surgical tool. The multi-positional grip may include a grip housing extending along an axis between a first end and a second end. A bottom surface may at least partially define a first grasping depression and a second grasping depression spaced from the first grasping depression. A top surface is spaced from the bottom surface by a pair of side surfaces. The top surface may include a user interface located proximate to the first end that may include at least one operational input.

In some implementations, the disclosure may provide for a surgical tool including a multi-positional grip. The multi-positional grip may include a grip housing extending along an axis between a first end that may be configured to be coupled to a surgical instrument and a second end that may be configured to be coupled to a control console. A bottom surface may at least partially define a first grasping depression proximate the first end and a second grasping depression proximate the second end. A top surface is spaced from the bottom surface by a pair of side surfaces. The top surface may define a third grasping depression and may further include a user interface that may be at least partially aligned with the first grasping depression along the axis and located between the first end and the third grasping depression.

In various implementations, the disclosure may provide for a multi-positional grip for a surgical tool. The multi-positional grip may include a grip housing extending along an axis between a first end that may be configured to couple to a surgical instrument and a second end. A bottom surface may at least partially define a first grasping depression proximate the first end and a second grasping depression proximate the second end that may be spaced from the first grasping depression. A top surface is spaced from the bottom surface by a pair of side surfaces. The top surface may include a user interface located proximate the first end and a third grasping depression proximate the second end may be at least partially axially aligned with the first grasping depression and the second grasping depression.

In some implementations, a multi-positional grip is configured to provide a plurality of holding positions by locating a provided caregiver's palm and hand, the hand including a thumb digit, an index finger, a long finger, a ring finger, and a pinky finger.

These and other features, objects and advantages of the present disclosure will become apparent upon reading the following description thereof together with reference to the accompanying drawings.

### BRIEF DESCRIPTION OF THE DRAWINGS

FIG. 1 is a top perspective view of a surgical tool with a multi-positional grip of an exemplary implementation in accordance with an aspect of the present disclosure;
FIG. 2 is a side profile view of a surgical tool with a multi-positional grip in accordance with an aspect of the present disclosure;
FIG. 3 is a top profile view of a surgical tool with a multi-positional grip o in accordance with an aspect of the present disclosure;
FIG. 4 is a bottom profile view of a surgical tool with a multi-positional grip in accordance with an aspect of the present disclosure;
FIG. 5A is a side profile view of a surgical tool illustrating relative sizes of various features of a multi-positional grip in accordance with an aspect of the present disclosure;
FIG. 5B is a first rear cross-sectional view of a surgical tool illustrating relative sizes of various features of a multi-positional grip in accordance with an aspect of the present disclosure;
FIG. 5C is a second rear cross-sectional view of a surgical tool illustrating relative sizes of various features of a multi-positional grip in accordance with an aspect of the present disclosure;
FIG. 5D is a pictorial flow chart diagram demonstrating multiple holding positions of a multi-positional grip of a surgical tool in accordance with an aspect of the present disclosure;
FIG. 6A is a side profile view of a surgical tool with a multi-positional grip of another exemplary implementation in accordance with an aspect of the present disclosure;
FIG. 6B is a side profile view of a surgical tool with a multi-positional grip of yet another exemplary implementation in accordance with an aspect of the present disclosure;
FIG. 6C is a side profile view of a surgical tool with a multi-positional grip of still yet another exemplary implementation in accordance with an aspect of the present disclosure;
FIG. 7A is a disassembled view of a surgical tool in the form of a shaver in accordance with an aspect of the present disclosure;
FIG. 7B is a projected assembly view of a surgical tool demonstrating a cutting window and housing in accordance with an aspect of the present disclosure;
FIG. 8 is a pictorial block diagram demonstrating a surgical tool and control system for the surgical tool in accordance with an aspect of the present disclosure; and
FIG. 9 is a top perspective view of a surgical tool with a cable attachment in accordance with an aspect of the present disclosure.

### DETAILED DESCRIPTION

In the following description, reference is made to the accompanying drawings, which show specific implementations that may be practiced. Wherever possible, the same reference numbers will be used throughout the drawings to refer to the same or like parts. It is to be understood that other implementations may be utilized and structural and functional changes may be made without departing from the scope of this disclosure.

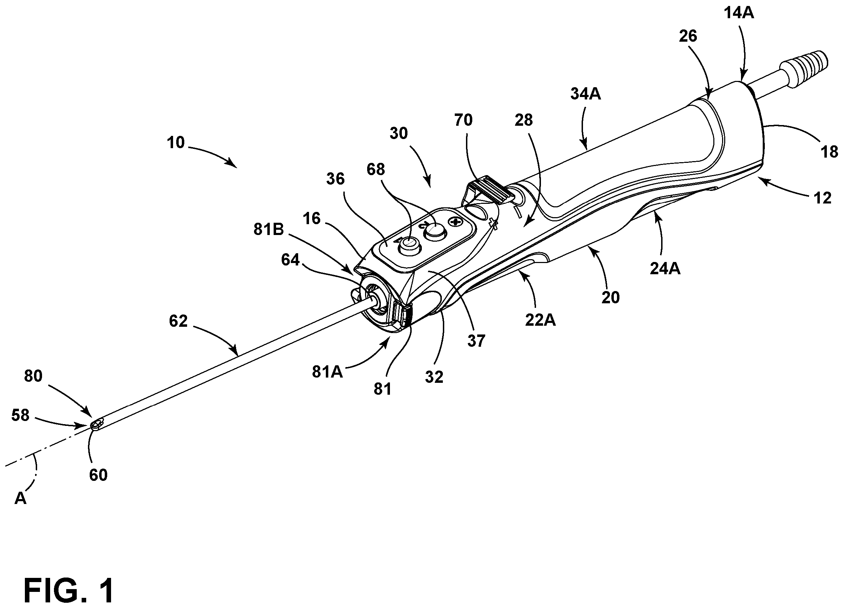

Referring generally to FIGS. 1-4, the disclosure provides for a variety of features and operating methods related to the handling of a surgical tool 10 between a variety of beneficial orientations. As demonstrated, the surgical tool 10 may include a multi-positional grip 12. The multi-positional grip 12 may include a grip housing 14A extending along an axis A between a first end 16 and a second end 18. A bottom surface 20 may at least partially define a first grasping depression 22A and a second grasping depression 24A that may be spaced from the first grasping depression 22A. A top surface 26 is spaced from the bottom surface 20 by a pair of side surfaces 28. The top surface 26 may include a user interface 30 located proximate the first end 16 that may include at least one operational input.

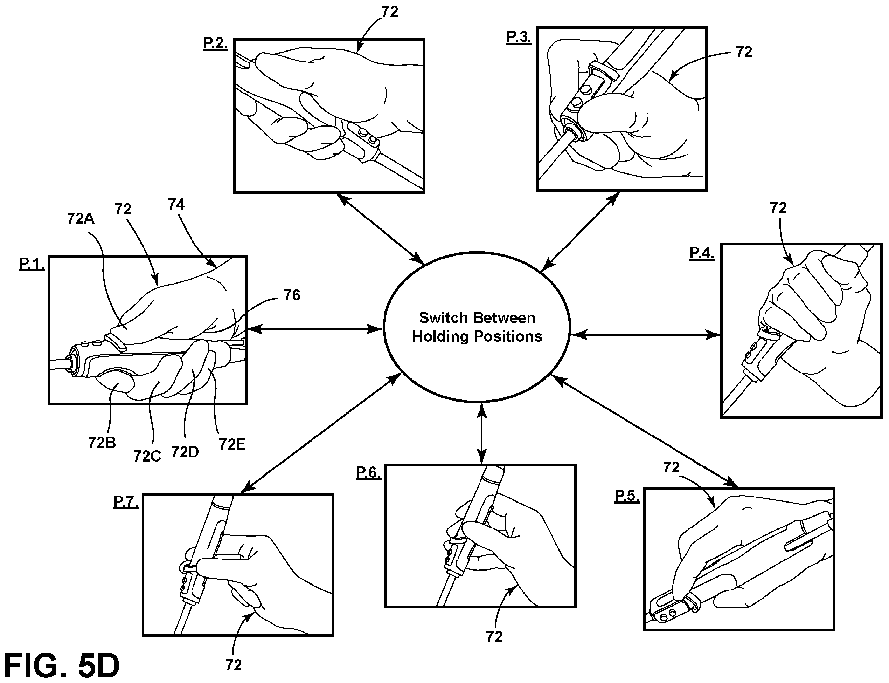

In general, the multi-positional grip 12 may provide various features (e.g., the first and second grasping depressions 22A, 24A), shapes, and materials, that improve handling and operation of the surgical tool 10. More particularly, the multi-positional grip 12 may allow a caregiver (e.g., an operator of the surgical tool 10) to grasp the various features to securely hold the surgical tool 10 at different orientations (FIG. 5D) during operation of the surgical tool 10. The flexibility provided by the different orientations can improve comfort based on caregiver preference and provide operational benefits for different procedures and/or at different stages of an operation.

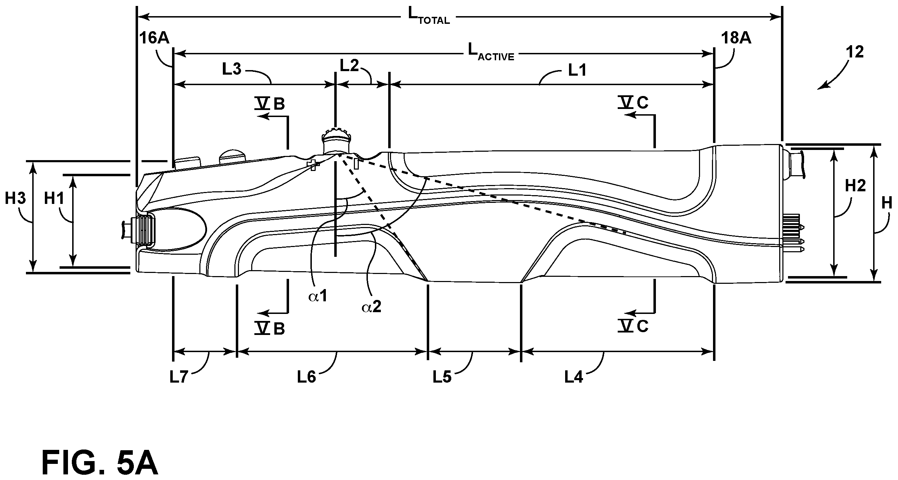

With continued reference to FIGS. 1-4, the pair of side surfaces 28 define a mean width and the bottom and top surfaces 20, 26 define a mean height that may be greater than the mean width W. For example, the mean width may vary along the axis A to facilitate various holding orientations and may generally conform to the corresponding dimensions of a hand of the caregiver to ensure comfort and security during usage. The following description provided in reference to FIGS. 1-4 introduces a variety of features that may beneficially facilitate the different holding orientations as discussed throughout the application. These features and the corresponding relationships among the features are later described in reference to FIGS. 5A-5D, which provides examples of proportions and relationships among the features that may facilitate the holding orientations and provide improved flexibility in usage of the multi-positional grip 12. By providing the features aligned relative to various operating inputs (e.g., of the user interface 30) or portions positioned along a length of the multi-positional grip 12, the surgical tool 10 may provide a variety of features that can be applied alone or in combination to improve the operation and flexibility of the surgical tool 10 to suit various applications and preferences.

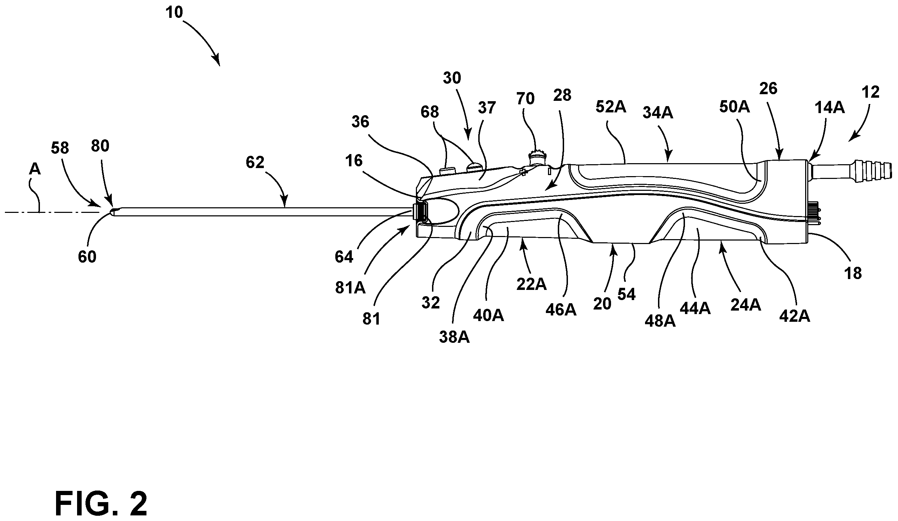

The engagement and flexibility of the multi-positional grip 12 may be supported by various features. In some implementations, a width between the side surfaces 28 may be the widest near the axis A and become smaller towards the bottom surface 20 and the side surface 28. The width proximate the top surface 26 may be smaller than the width proximate the bottom surface 20. In some implementations, a height between the bottom and top surfaces 20, 26 may become larger from the first end 16 to the second end 18. The first and second grasping depressions 22A, 24A may be bounded by a protruding surface 32 extending from the bottom surface 20 along part of the side surfaces 28 that may terminate before the first end 16. In some implementations, the top surface 26 may at least partially define a third grasping depression 34A that may be proximate the second end 18 and may be at least partially aligned with the second grasping depression 24A (e.g., completely and/or substantially overlap) and the first grasping depression 22A along the axis A (e.g., partially overlap). As best depicted in FIG. 2, the top surface 26 may be substantially flat (e.g., with the exception of the third grasping depression 34A) until an interface portion 36 of the top surface 26 that supports the user interface 30. The interface portion 36 may be angled towards the axis A in a direction towards the first end 16. The interface portion 36 may be substantially flat. A pair of angled grasping surfaces 37 may be defined by the side surfaces 28. The angled grasping surfaces 37 may be substantially flat and become closer in a direction of the interface portion 36. The first grasping depression 22A may be at least partially aligned with the interface portion 36 along the axis A. The height may generally become progressively larger from the first end 16 towards the second end 18 along the bottom surface 20 except for the first and second grasping depressions 22A, 24A.

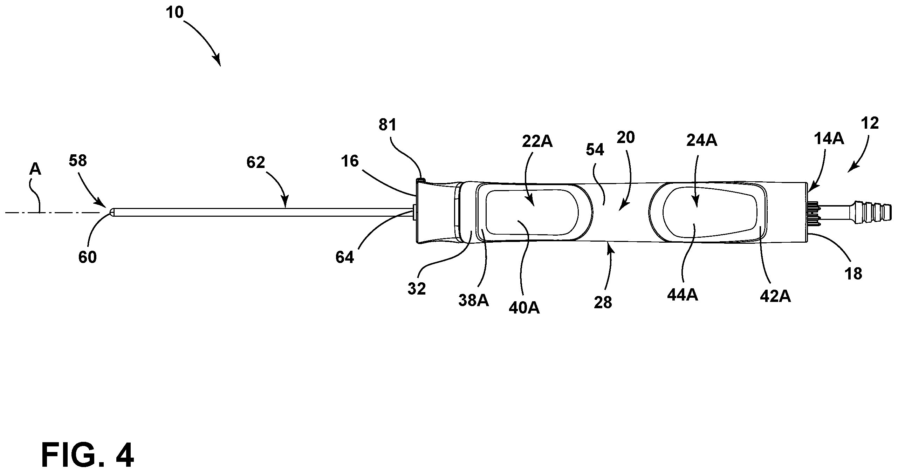

With continued reference to FIGS. 1-4, the first grasping depression 22A may be defined by a first depression wall 38A and a first depression floor 40A (FIGS. 2 and 4). The first depression wall 38A may generally extend towards the axis A to the first depression floor 40A to define a first depth. The first depression wall 38A may be symmetrical with respect to and along the side surfaces 28 along the axis A (FIG. 4) for both left-hand and right-hand operation. Similarly, the second grasping depression 24A may be defined by a second depression wall 42A and a second depression floor 44A (FIGS. 2 and 4). The second depression wall 42A may extend towards the axis A to the second depression floor 44A to define a second depth. The second depression wall 42A may be symmetrical with respect to and along the side surfaces 28 along the axis A (FIG. 4) for both left-hand and right-hand operation to allow both left and right-handed caregivers to comfortably and securely grasp the multi-positional grip 12 at different orientations. In some implementations, the first depth is approximately equal (e.g., substantially equal and/or equal) to the second depth.

Referring to FIGS. 2-4, the first grasping depression 22A may be located proximate to the first end 16, and the first depression wall 38A is at least partially defined by the pair of side surfaces 28. Likewise, the first depression floor 40A may be at least partially defined by the pair of side surfaces 28. In some implementations, the first depression wall 38A may include a pair of first longitudinal portions 46A extending between the first end 16 and the second end 18 (FIG. 2). The first longitudinal portions 46A may be angled from the bottom surface 20 towards the top surface 26 in a direction towards the second end 18. The second grasping depression 24A may be located proximate the second end 18 and the second depression wall 42A is at least partially defined by the pair of side surfaces 28. Likewise, the second depression floor 44A may be at least partially defined by the pair of side surfaces 28. In some implementations, the second depression wall 42A may include a pair of second longitudinal portions 48A extending between the first end 16 and the second end 18 (FIG. 2). The second longitudinal portions 48A may be angled from the bottom surface 20 towards the top surface 26 in a direction towards the first end 16.

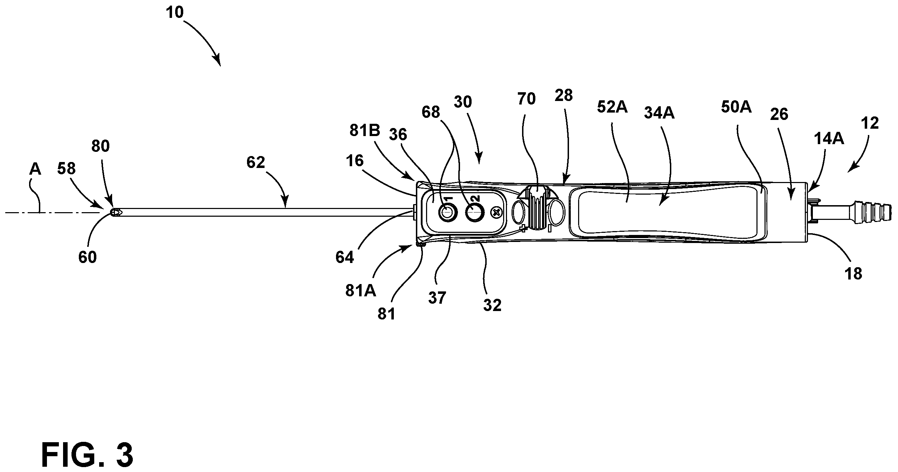

The third grasping depression 34A may be defined by a third depression wall 50A and a third depression floor 52A (FIGS. 2 and 3). The third depression wall 50A may extend towards the axis A to the third depression floor 52A to define a third depth. The third depression wall 50A may be symmetrical with respect to and along the side surfaces 28 along the axis A (FIG. 3) for both left-hand and right-hand operations. In some implementations, the third depth is equal to, substantially equal to, less, or greater than the first depth and/or the second depth. The third grasping depression 34A may be located proximate the second end 18 and the third depression wall 50A may be at least partially defined by the pair of side surfaces 28. Likewise, the third depression floor 52A may be at least partially defined by the pair of side surfaces 28.

With continued reference to FIGS. 2-4, the first depression floor 40A, the second depression floor 44A, and/or the third depression floor 52A may be curved about the axis A. In some implementations, the first end 16 and the second end 18 define a total length donated as L_{TOTAL} therebetween and the first grasping depression 22A mirrors (e.g., in shape and/or size) the second grasping depression 24A along the total length L_{TOTAL} (FIG. 5A). The second grasping depression 24A may be spaced from the first grasping depression 22A by a bridge portion 54 (e.g., defined by the protruding surface 32).

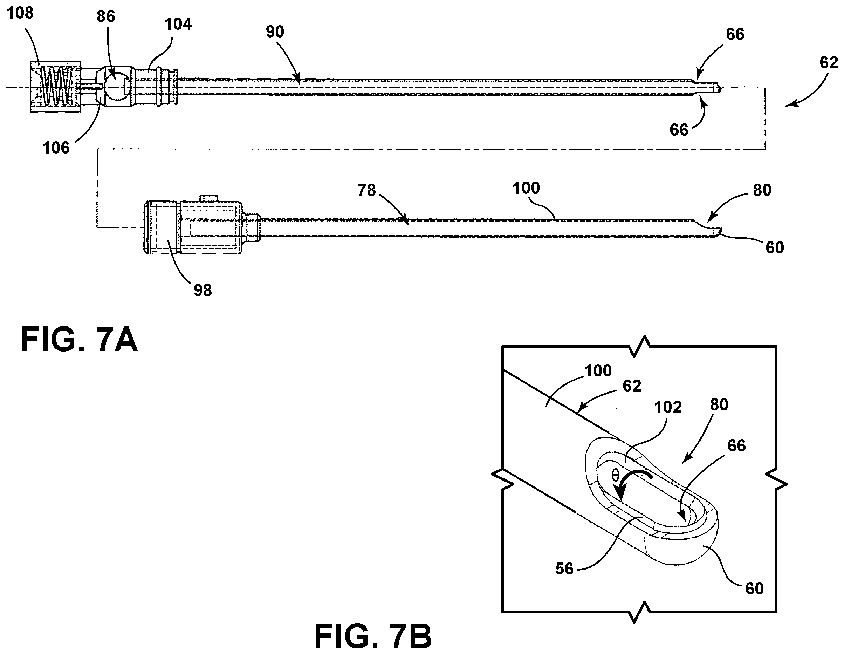

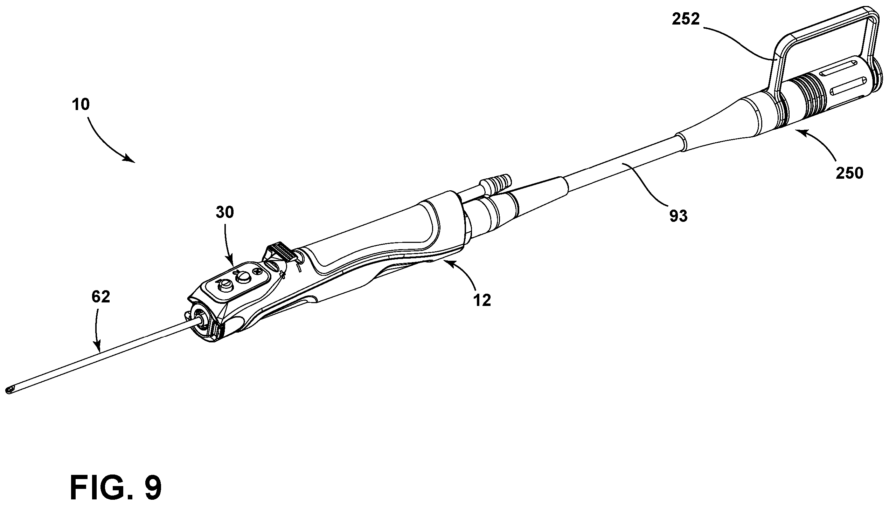

The exemplary surgical tool 10 described throughout the application is depicted as including a surgical instrument that has a cutting tool and, more particularly, a rotary cutting tool. However, surgical tools with surgical instruments other than cutting tools or rotary cutting tools may be implemented with the multi-positional grip 12 in scenarios where it is beneficial to have the flexibility provided by the different orientations to improve comfort based on caregiver preference, caregiver handedness, and provide operational benefits for different procedures and/or at different stages of an operation. More particularly, the surgical instrument may correspond to shavers, burrs, power rasps, drills, picks, biters, electrosurgical or radio frequency probes, other surgical operational tools, and/or the like. For example, the surgical instrument may correspond to a variety of different forms of rotary cutting tools having one or more rotary cutting members 56 (e.g., blades, cutting windows, burrs, etc.) that may form a cutting head 58 of the surgical tool 10 (FIG. 7B). As shown, the cutting head 58 may be disposed at a distal end portion 60 of an elongated probe 62 extending from a proximal end portion 64 in connection with the grip housing 14A. In this configuration, the cutting head 58 may be formed by at least one cutting window 66 as further discussed in reference to FIGS. 7A and 7B. As described throughout the disclosure, the rotation or oscillation of the cutting window 66 may be adjusted by the caregiver via the user interface 30 to selectively implement various cutting routines or sequences to provide a desired cutting operation. In various implementations as further discussed in reference to FIG. 9, the surgical tool 10 (e.g., the user interface 30) may be in communication with a control system 200 that is configured to control a rotational position θ of the one or more rotary cutting members 56 and further implement a variety of rotational cutting routines that may control a series or sequence of oscillations of the rotary cutting members 56. The at least one operational input may include one or more buttons 68 and an aspiration control interface or input 70 (e.g., a toggle that may be pivotal along the axis A) for communicating with the control system 200 and implementing various operational protocols of the surgical tool 10 as further discussed in reference to FIG 9. The buttons 68 may be located on the interface portion 36 and the aspiration input 70 may be located between the interface portion 36 and the third grasping depression 34A.

The multi-positional grip 12 may be at least partially covered (e.g., formed, layered, etc.) by a non-slip material. For example, rubbers, foams, microcrystalline aluminum oxide coating, non-slip textures, combinations thereof, the like, and/or other medical grade non-slip materials that can repeatedly undergo sterilization without degradation. The non-slip material may cover the first grasping depression 22A (e.g., the first depression wall 38A and/or the first depression floor 40A), the second grasping depression 24A (e.g., the second depression wall 42A and/or the second depression floor 44A), and/or the third grasping depression 34A (e.g., the third depression wall 50A and/or the third depression floor 52A). In some implementations, the non-slip material may cover additional portions of the multi-positional grip housing 14A (e.g., the bottom surface 20, the top surface 26, and/or the side surfaces 28). In some implementations, the non-slip material may entirely cover and/or cover substantially the whole multi-positional grip housing 14A. For example, in some implementations, the non-slip material may cover the entire multi-positional grip housing 14A except for the user interface 30 (e.g., the interface portion 36 and a surface of the multi-positional grip housing 14A that interfaces with the aspiration input 70). In some implementations, the non-slip material may also not cover operable surfaces of the multi-positional grip housing 14A that interface with proximal end portion 64 of the surgical tool (e.g., portions of the first end 16) and portions of the second end 18 that operably connect to, for example, control systems, power systems, vacuum systems, and liquid introduction or recovery systems.

In some implementations, the non-slip material may be textured, exhibiting a textured grit. For example, the grit size (e.g., on average) may be between about 25 and 1000 microns, for example, 25 or more microns, 50 or more microns, 100 or more microns, 200 or more microns, 300 or more microns, 400 or more microns, 500 or more microns, 600 or more microns, 700 or more microns, 800 or more microns, 900 or more microns, about 1000 microns, 1000 or less microns, 900 or less microns, 800 or less microns, 700 or less microns, 600 or less microns, 500 or less microns, 400 or less microns, 300 or less microns, 200 or less microns, 100 or less microns, 50 microns or less, 25 microns or less, between 200 microns and 400 microns, between 300 microns and 400 microns, between 350 microns and 400 microns, between 200 microns and 250 microns, between 50 microns and 100 microns, between 25 microns and 50 microns, between 10 microns and 25 microns, about 373 microns, about 217 microns, about 86 microns, about 35 microns, or about 16 microns. The textured grit may be produced, for example, by bead blasting various surfaces of the multi-positional grip housing 14A with grit aluminum oxide (e.g., 16 grit), at about 50-60 PSI. In this manner, a caregiver can securely grasp the multi-positional grip housing 14A in a variety of orientations and holding positions without slippage.

As shown in FIG. 5A, a side profile view of the surgical tool 10 illustrating relative and absolute sizes of various features of a multi-positional grip 12 is depicted. These relative and absolute sizes may be configured to provide comfort, flexibility, and ergonomics to a standard hand size for a majority of caregivers. Further, it should be appreciated that the absolute sizes may be adjusted while the relative sizes may remain the same for standard hand ratios of varying sizes. More particularly, the total length L_{TOTAL} may be defined by the distance between the first end 16 and the second end 18. It should be appreciated that the total length L_{TOTAL} and other lengths described herein are defined as the distance between points axially (e.g., linearly). In some implementations, the total length L_{TOTAL} is between about 125mm to about 200mm, for example between 150mm and 180mm, between 160mm and 175mm, or about 165mm. It should be appreciated that the total length L_{TOTAL} may be varied above or below the above ranges but may generally provide additional surfaces for holding by the caregiver during use. An active length L_{ACTIVE} may be defined as the portion of the multi-positional grip housing 12 that may include the first grasping depression 22A, the second grasping depression 24A, the third grasping depression 34A, and the user interface 30 (e.g., the button 68 most proximate the first end 16). In some implementations, the active length L_{ACTIVE} is between about 100mm to about 200mm, for example between 125mm and 175mm, between 130mm and 160mm, or about 140mm. The total length L_{TOTAL} may include portions extending from the active length L_{ACTIVE} to the second end 18 and the first end 16 (e.g., between about 5% and 20% of the active length L_{ACTIVE}). The active length L_{ACTIVE} extends between a first active end 16A and a second active end 18A.

The third grasping depression 34A may extend from the second active end 18A towards the first active end 16A a first minor length L1. The first minor length L1 may be about 60% (e.g., between about 50% and 70%) of the active length L_{ACTIVE} or between about 80mm and 90mm. The user interface 30 (e.g., a center of the aspiration input 70) may be spaced from the third grasping depression 34A a second minor length L2. The second minor length L2 may be about 10% of the active length L_{ACTIVE} or between about 10mm and 15mm. The user interface 30 of the multi-positional grip 12 may extend from the first active end 16A a third minor length L3. The third minor length L3 may be about 30% (e.g., between about 20% and 40%) of the active length L_{ACTIVE} or between about 40mm and 50mm. In some implementations, a portion of the third grasping depression 34A on the second active end 18A to the aspiration input 70 (e.g., L1 + L2) is about 75% (e.g., between about 60% and 85%) of the active length L_{ACTIVE} or between about 80mm and 120mm. In some implementations, a center of the third grasping depression 34A is spaced (e.g., along the axis A) from the aspiration input 70 about 40% (e.g., between about 35% and 45%) of the active length L_{ACTIVE} or about 50mm to 60mm.

With continued reference to FIG. 5A, on the bottom surface 20 and from the second active end 18A, the second grasping depression 24A may extend from the first active end 16A a fourth minor length L4. The fourth minor length L4 may be about 35% (e.g., between about 30% and 40%) of the active length L_{ACTIVE}. At the second active end 18A a portion of the first grasping depression 22A and a portion of the third grasping depression 34A are aligned along the axis A to form a grasping wedge between opposing depression walls 42A, 50A, preventing forward (e.g., in a direction along the axis A towards the first end 16) slippage of the multi-positional grip 12 during usage. The fourth minor length L4 may be greater than 50% (e.g., between about 55% and 65%) the first minor length L1 defined by the third grasping depression 34A, about 35% (e.g., between about 30% and 40%) of the active length L_{ACTIVE}, or between about 45mm and 55mm. In some implementations, a center of the second grasping depression 24A is spaced (e.g., along the axis A) from the aspiration input 70 about 65% (e.g., between about 60% and 70%) of the active length L_{ACTIVE} or about 85mm to 95mm. In some implementations, a portion of the second grasping depression 24A nearest the first active end 16A is spaced (e.g., along the axis A) from the aspiration input 70 about 30% (e.g., between about 25% and 35%) of the active length L_{ACTIVE} or about 45mm to 55mm.

The bridge portion 54 may extend between the second grasping depression 24A and the first grasping depression 22A a fifth minor length L5. The fifth minor length L5 may be about 15% (e.g., between about 10% and 20%) of the active length L_{ACTIVE}. In some implementations, the fifth minor length L5 terminates in axial alignment with the third grasping depression 34A and, more particularly, proximate where the third depression wall 50A and the third depression floor 52A meet proximate the first active end 16A. The first and second depression walls 38A, 42A bounding the bridge portion 54 form opposing grasping surfaces a wedge that may further prevent forward and backward slippage of the multi-positional grip 12 during usage. In some implementations, a center of the bridge portion 54 is spaced (e.g., along the axis A) from the aspiration input 70 about 30% (e.g., between about 25% and 35%) of the active length L_{ACTIVE} or about 35mm to 45mm.

The first grasping depression 22A may extend from the bridge portion 54 toward the first active end 16A a sixth minor length L6. The sixth minor length L6 may be equal to the fourth active length L4 or greater than 50% (e.g., between about 55% and 65%) the first minor length L1 defined by the third grasping depression 34A, about 35% (e.g., between about 30% and 40%) of the active length L_{ACTIVE}, or between about 45mm and 55mm. In this manner, the third depression wall 50A closest to the first active end 16A may oppose and axially overlap the first depression wall 38A closest the second active end 18A, which may act as another grasping wedge during usage for further prevention of slippage. In addition, the aspiration input 70 may be located substantially centrally (e.g., within 10% of the active length L_{ACTIVE}) relative to the axis A along the sixth minor length L6. Stated generally, the first depression 22A may be at least partially aligned with the aspiration input 70 along the axis A. In some implementations, a portion of the first grasping depression 22A nearest the second active end 18A is spaced (e.g., along the axis A) from the aspiration input 70 about 20% (e.g., between about 15% and 25%) of the active length L_{ACTIVE} or about 25mm to 35mm.

A remaining portion of the multi-positional grip 12 may extend from the first grasping depression 22A to the first active end 16A a seventh minor length L7. The seventh minor length L7 may be about 15% (e.g., between about 10% and 20%) of the total length L_{TOTAL}. The protruding surface 32 may terminate along the seventh minor length L7 (e.g., centrally) and provide further grasping surfaces. A portion of the first depression wall 38A (e.g., nearest the second active end 18A) may include a generally linear portion that may extend towards the aspiration input 70 donated as α1. Likewise, a portion of the second depression wall 42A (e.g., the second longitudinal portions 48A) may include a linear portion that may extend towards the aspiration input 70 donated as α2. In this manner, the caregiver may be generally guided toward the aspiration input 70 without necessarily requiring visual confirmation.

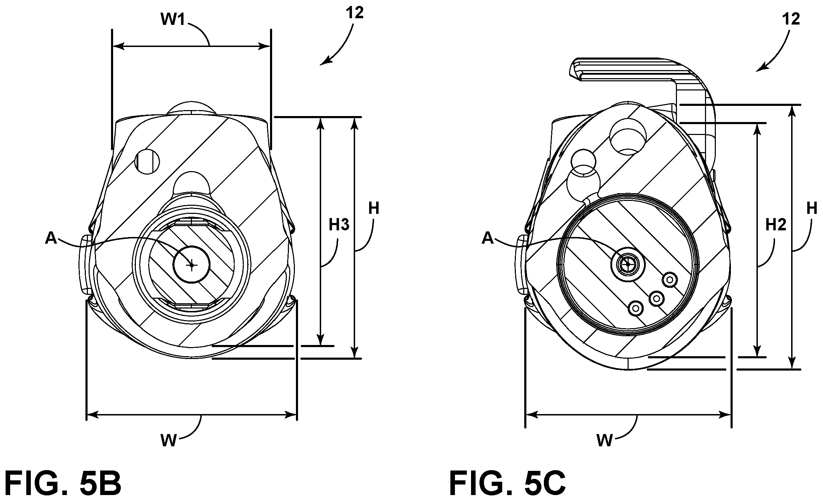

With reference now to FIGS. 5A-5C, a height H or maximum height may be defined by the distance between the bottom surface 20 and the top surface 26. It should be appreciated that the maximum height H and other heights described herein are defined as linear distances between the bottom surface 20 and the top surface 26 (e.g., perpendicular to the axis A). In some implementations, the maximum height is proximate the second end 18. The maximum height may be between about 25mm to about 45mm, for example between 30mm and 40mm, or about 35mm. A first minor height H1 may be defined between the bottom surface 20 and the top surface 26 proximate the first end 16. The first minor height H1 may be about 70% of the maximum height H. A second minor height H2 may be defined between the bottom surface 20 and the top surface 26 and, more particularly, between the third depression floor 52A and the second depression floor 44A (FIG. 5C). The second minor height H2 may be between about 80% and about 90% of the maximum height H. A third minor height H3 may be defined between the bottom surface 20 and the top surface 26 and, more particularly, between the interface portion 36 and the first depression floor 40A (FIG. 5B) and/or bottom surface 20. The third minor height H3 may be between about 70% and about 80% of the maximum height H (e.g., less than the second minor height H2).

In FIGS. 5B and 5C, a width W or maximum width may be defined by the distance between the side surfaces 28. It should be appreciated that the maximum width W and other widths described herein are defined as linear distances between the side surfaces 28 (e.g., perpendicular to the axis A and the height H). In some implementations, the maximum width W is proximate the second end 18. The maximum width may be between about 20mm to about 30mm, for example between 22mm and 28mm, or about 25mm (e.g., less than the maximum height H). A first minor width W1 may be defined between the side surfaces 28 over the interface portion 36. The first minor width W1 may be about 70% the maximum width W. As depicted in FIG. 5B and 5C, the first depression floor 40A and the second depression floor 44A may be curved. In some implementations, at least a portion (e.g., a minority or a majority) of the first depression floor 40A and/or the second depression floor 44A may be defined by a common curvature about the axis A (e.g., a curvature or circumference formed by the same size or substantially the same size radius or radii). However, it should be appreciated that in some implementations, the first depression floor 40A and/or the second depression floor 44A may be defined by different curvatures. As depicted in FIG. 5C the third depression floor 52A may be curved. In some implementations, the third depression floor 52A may be arched (e.g., defined by two or more radii) and symmetrical relative to both side surfaces 28 and with respect to the axis A.

Referring now to FIG. 5D, a pictorial flow chart diagram demonstrates multiple holding or grasping positions of the multi-positional grip 12 that can be utilized while operating the surgical tool 10. As a matter of reference, a typical caregiver will generally utilize their hands 72, wrist 74, and arms during operation of the surgical tool 10. The typical hand 72 includes a thumb digit 72A (e.g., opposing thumb), fingers (e.g., opposing fingers) (i.e., an index finger 72B, a middle digit or long finger 72C, ring finger 72D, a pinky finger 72E), and a palm 76. The relative and absolute sizes depicted and described in reference to FIGS. 5A-5C guide a caregiver's fingers, thumb digit and palm into different ones of the grasping depressions 22A, 24A, 34A during usage and in multiple holding orientations. As will be appreciated in reference to the holding orientations depicted in FIG. 5D, the thumb digit 72A and/or one of the fingers 72B-72E may be proximate and engageable with the aspiration input 70 and/or buttons 68 regardless of the holding position utilized to utilize the user interface 30 during usage.

A first holding position is designated by reference P.1. and will be referred to herein as a forward holding position. In the forward holding position P.1., the multi-positional grip 12 may be in a supine orientation (e.g., generally horizontal with the top surface 26 facing upwardly). The thumb digit 72A may grasp the top surface 26 (e.g., proximate or over the user interface 30) and one or more opposing fingers (e.g., one opposing finger, two or more opposing fingers, three or more opposing fingers, or four opposing fingers) may grasp the bottom surface 20. In the forward holding position P.1., the surgical instrument (e.g., the elongated probe 62) may proximate the thumb digit 72A and the index finger 72B and extend generally away from the caregiver. The thumb digit 72A may be aligned with the user interface 30 (e.g., engageable with the aspiration input 70 and/or buttons 68), a thumb portion of the palm 76 may rest in the third grasping depression 34A, and the opposing fingers may wrap around and in engagement with the bottom surface 20. More particularly, a first opposing finger (e.g., the index finger 72B) may be located in the first grasping depression 22A, a second opposing finger (e.g., the pinky finger 72E) may be located in the second grasping depression 24A, and a third and/or fourth opposing finger (e.g., the long finger 72C and/or the ring finger 72D) may be located in the first and second grasping depression 22A, 24A, respectively, and/or on the bridge portion 54. The forward holding position P.1. may be beneficial for moving the surgical instrument along the axis A in a substantially horizontal orientation and articulating the axis A to an optimal operational angle in a forward direction away from the caregiver while having the user interface 30 visible to the caregiver.

A second holding position is designated by reference P.2. and will be referred to herein as a backward holding position. In the backward holding position P.2., the multi-positional grip 12 may be in the supine orientation. The surgical instrument (e.g., the elongated probe 62) is proximate to the pinky finger 72E and may extend generally away from the caregiver. The thumb digit 72A may rest along the top surface 26 (e.g., in the third grasping depression 34A and/or proximate the second end 18) and a thumb portion of the palm 76 may rest proximate the user interface 30 and/or first end 16. The one or more opposing fingers (e.g., one opposing finger, two or more opposing fingers, three or more opposing fingers, or four opposing fingers) may wrap around and may be in engagement with the bottom surface 20. More particularly, a first and/or second opposing finger (e.g., the index finger 72B and/or the long finger 72C) may be rested in the second grasping depression 24A, while a third and/or fourth opposing finger (e.g., the ring finger 72D and/or pinky finger 72E) may be rested in the first grasping depression 22A. In the backward holding position P.2., a tip of the ring finger 72D may be aligned with the user interface 30. The backward holding position P.2. may be beneficial for moving the surgical instrument along the axis A in a substantially horizontal orientation and articulating the axis A to an optimal operational angle in a backward direction towards the caregiver.

A third holding position is designated by reference P.3. and will be referred to herein as a tripod holding position. In the tripod holding position P.3., the multi-positional grip 12 may be in an angled orientation (e.g., generally between an upright orientation and the supine orientation). The user interface 30 may generally face away from the caregiver. The surgical instrument (e.g., the elongated probe 62) and/or first end 16 may proximate the thumb digit 72A and an opposing finger (e.g., the index finger 72B or long finger 72C) and extend downwardly. The thumb digit 72A and the opposing finger may be aligned on opposite sides of the user interface 30 (e.g., engageable with the aspiration input 70 and/or buttons 68), squeezing portions of the angled side surfaces 37 proximate the interface portion 36. A portion of the palm 76 between the thumb digit 72A and index finger 72B may rest in the first grasping depression 22A. The tripod holding position P.3. may be beneficial for moving the surgical instrument along the axis A and articulating the axis A to an optimal operational angle in a downward and angled direction away from or towards the caregiver.

A fourth holding position is designated by reference P.4. and will be referred to herein as a palmer holding position. In the palmer holding position P.4., the multi-positional grip 12 may be in an angled orientation (e.g., generally between an upright orientation and the supine orientation). The user interface 30 may generally face away from the caregiver. The surgical instrument (e.g., the elongated probe 62) is proximate to the pinky finger 72E and may extend downwardly. One or more fingers (e.g., one opposing finger, two or more opposing fingers, three or more opposing fingers, or four opposing fingers) may wrap around and may be in engagement with the top surface 26 while the thumb digit 72A may wrap around the bottom surface 20. More particularly, the index finger 72B, long finger 72C, and ring finger 72D may be located in the third grasping depression 34A, while the pinky finger 72E may be located proximate the user interface 30 (e.g., engageable with the aspiration input 70 and/or buttons 68). A portion of the palm 76 between the thumb digit 72A and index finger 72B may rest in the second grasping depression 24A. The palmer holding position P.4. may be beneficial for moving the surgical instrument along the axis A and articulating the axis A to an optimal operational angle in a downward and angled direction with respect to the caregiver.

A fifth holding position is designated by reference P.5. and will be referred to herein as a digital pronate holding position. In the digital pronate holding position P.5., the multi-positional grip 12 may be in a prone orientation (e.g., generally horizontal with the top surface 26 facing downwardly) or rotatable along the axis A between the prone orientation and the supine orientation. The surgical instrument (e.g., the elongated probe 62) is proximate to the thumb digit 72A and the index finger 72B and may extend away from the caregiver. An opposing finger (e.g., the index finger 72B or the long finger 72C) may be rested on or proximate one of the angled side surfaces 37 of the interface portion 36 (e.g., engageable with the aspiration input 70 and/or buttons 68) and the thumb digit 72A may be rested on a side surface 28 proximate the top surface 26. A portion of the palm 76 between the thumb digit 72A and index finger 72B may rest in the second grasping depression 24A. The digital pronate holding position P.5. may be beneficial for moving the surgical instrument along the axis A, turning the surgical instrument about the axis A, and articulating the axis A to an optimal operational angle in a forward direction away from the caregiver.

A sixth holding position is designated by reference P.6. and will be referred to herein as a digital holding position. In the digital holding position P.6., the multi-positional grip 12 may be in an angled or upright orientation. The user interface 30 may generally face away from or towards the caregiver. One or more opposing fingers (e.g., the long finger 72C, the index finger 72D, and/or the pinky finger 72E) may be located proximate to and engageable with the aspiration input 70 and/or buttons 68. The surgical instrument (e.g., the elongated probe 62) may proximate the pinky finger 72E and extend downwardly. The thumb digit 72A may be located on one of the side surfaces 28 (e.g., centrally and/or aligned with the bridge portion 54) and at least three opposing fingers (e.g., three or four opposing fingers) may be aligned on an opposite side surface 28. The palm 76 may be spaced from the multi-positional grip 12. The digital holding position P.6. may be beneficial for moving the surgical instrument along the axis A in upward and downward motions and articulating the axis A to an optimal operational angle from the caregiver.

A seventh holding position is designated by reference P.7. and will be referred to herein as a jaw chuck holding position. In the jaw chuck holding position P.7., the multi-positional grip 12 may be in an angled or upright orientation. The user interface 30 may generally face away from or towards the caregiver. The surgical instrument (e.g., the elongated probe 62) may be proximate to one or two opposing fingers (e.g., the index finger 72B and/or the long finger 72C) and the thumb digit 72A and extend downwardly. The thumb digit 72A may be located on one of the side surfaces 28 (e.g., centrally and/or aligned with the bridge portion 54) and the one or two opposing fingers may be aligned on an opposite side surface 28. One or more opposing fingers (e.g., the index finger 72B and/or the long finger 72C) may be located proximate to and engageable with the aspiration input 70 and/or buttons 68. The palm 76 may be spaced from the multi-positional grip 12. The jaw chuck holding position P.7. may be beneficial for moving the surgical instrument along the axis A in upward and downward motions and articulating the axis A to an optimal operational angle from the caregiver.

The holding positions P.1.-P.7. provide flexibility to the caregiver and allow the caregiver to select one of the holding positions P.1.-P.7. based on preference and operation parameters regardless of the caregiver handedness. In addition, any of the holding positions P.1.-P.7. can be switched to any of the other holding positions P.1.-P.7. based on immediate need throughout the operation. Various operations require insertion of the surgical instrument (e.g., the elongated probe 62) into a body of the patient such that the cutting head 58 is not visible while in use. The blind nature of these various operations requires adept usage of surgical implements and tools that vary widely among procedures and the corresponding diverse surgical implements and apparatuses. The various shapes and features of the multi-positional grip 12 can also be used for indicating a position of the cutting members 56, which may correspond to the top surface 26 of the grip housing 14A and the user interface 30. In addition, the holding positions P.1.-P.7. allow a caregiver to securely hold the multi-positional grip 12 with the cutting members 56 at a variety of orientation about the axis A for operating on bodily portions of a patient from above, below, and/or side surfaces. Further, many operations require prolonged usage of the surgical tool 10, as such, different holding positions P.1.-P.7. can be utilized to reduce stress on the hand 72, the wrist 74, and the arms of the caregiver by allowing different motions. In addition, the symmetrical shape may permit similar usage and flexibility for both left-handed and right-handed caregivers.

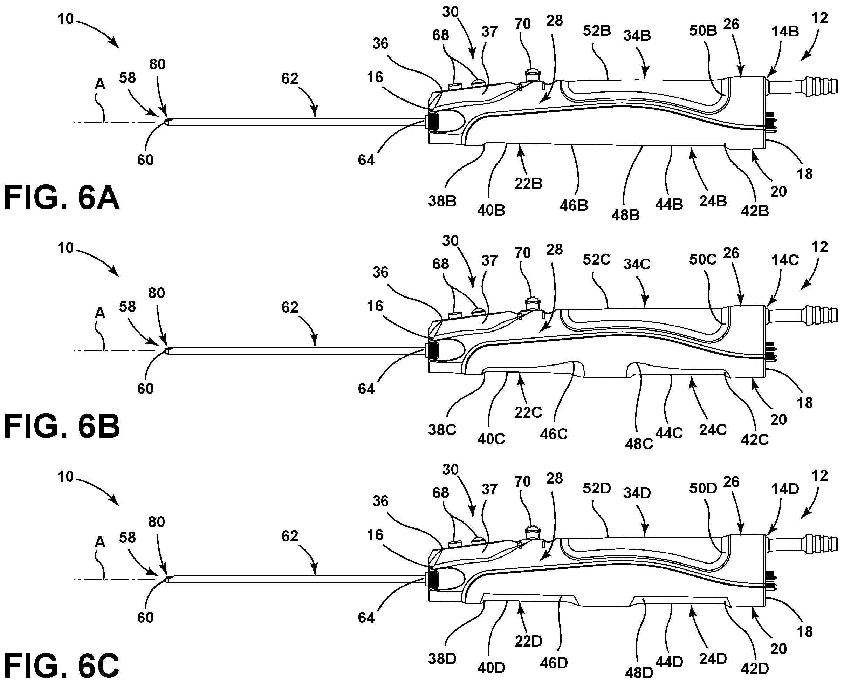

With reference now to FIGS. 6A-6C, various alternative constructions of the grip housing 14A are depicted. More particularly, FIG. 6A depicts a grip housing 14B of another exemplary implementation, FIG. 6B depicts a grip housing 14C of yet another exemplary implementation, and FIG. 6C depicts a grip housing 14D of still yet another exemplary implementation. Unless expressly stated otherwise, the grip housings 14B-14D depicted in FIGS. 6A-6C may share the same or similar features, shapes, relative sizes, absolute sizes, functionality, holding positions, and materials as the grip housing 14A.

In FIG. 6A, the grip housing 14B may include a first grasping depression 22B defined by a first depression wall 38B and a first depression floor 40B, a second grasping depression 24B defined by a second depression wall 42B and a second depression floor 44B, and a third grasping depression 34B defined by a third depression wall 50B and a third depression floor 52B. As depicted, the first grasping depression 22B and the second grasping depression 24B are not spaced from one another and merge together as a singular depression 22B, 24B. The combination of the first grasping depression 22B and the second grasping depression 24B may extend along the total length L_{TOTAL} a greater distance than the third grasping depression 34B. The first grasping depression 22B and the second grasping depression 24B may be defined substantially by the bottom surface 20 and the third grasping depression 34B may be defined substantially by the top surface 26. The first depression wall 38B may include a pair of first longitudinal portions 46B and the second depression wall 42B may include a pair of second longitudinal portions 48B aligned with the first longitudinal portions 46B and extending linearly between the first end 16 and the second end 18.

Referring now to FIG. 6B, the grip housing 14C may include a first grasping depression 22C defined by a first depression wall 38C and a first depression floor 40C, a second grasping depression 24C defined by a second depression wall 42C and a second depression floor 44C, and a third grasping depression 34C defined by a third depression wall 50C and a third depression floor 52C. As depicted, the first depression wall 38C may include a pair of first longitudinal portions 46C extending between the first end 16 and the second end 18. The first longitudinal portions 46C may be curved from a central region of the first longitudinal portions 46C from the bottom surface 20 towards the top surface 26 in a direction towards the second end 18. In some implementations, the second depression wall 42C may include a pair of second longitudinal portions 48C extending between the first end 16 and the second end 18. The second longitudinal portions 48C may be curved from a central region of the second longitudinal portions 48C from the bottom surface 20 towards the top surface 26 in a direction towards the first end 16.

In FIG. 6C, the grip housing 14D may include a first grasping depression 22D defined by a first depression wall 38D and a first depression floor 40D, a second grasping depression 24D defined by a second depression wall 42D and a second depression floor 44D, and a third grasping depression 34D defined by a third depression wall 50D and a third depression floor 52D. As depicted, the first depression wall 38D may include a pair of first longitudinal portions 46D and the second depression wall 42D may include a pair of second longitudinal portions 48D. Both the first and second longitudinal portions 46D, 48D may extend linearly between the first end 16 and the second end 18. In some implementations, the first and second longitudinal portions 46D, 48D may be perpendicular to one another. The first and second longitudinal portions 46D, 48D may be perpendicular to the bottom surface 20.

With reference now to FIGS. 7A-8, the probe 62 may include an interior passage 78 extending from the proximal end portion 64 to the distal end portion 60 and the distal end portion 60 may form an opening 80. A rotary member (e.g., one or more rotary cutting members 56) may extend through the interior passage 78 and in connection with a motor 82 and form the at least one cutting window 66. The rotary member may rotate within the interior passage 78 selectively positioning the at least one cutting window 66 in alignment with the opening 80. To effectuate the cutting operations or sequences of cutting routines, the control system 200 may communicate with a motor drive circuit 84 of the surgical tool 10 that may be configured to control the motor 82 to selectively position the one or more rotary cutting members 56 to specified rotational positions θ in rapid succession. In such cases, a cutting routine may be adjusted to rotate the one or more cutting members 56 over rotational ranges of the rotational position θ, which may control a duty cycle between different cutting styles or levels of refinement or aggressiveness of cut to control the operation of the rotary surgical tool 10. In some cases, the motor drive circuit 84 and motor 82 may operate in concert to control the rotational position θ to increments of 5°, 3°, 1°, or even higher levels of precision depending on the specific use case and application for the surgical tool 10. In this way, cutting routines and operations supported by the surgical tool 10 and the control system 200 may provide for improved operation to implement a variety of control routines, some of which are described in the following detailed description.

The surgical tool 10 may connected to a surgical pump 88 or vacuum pump that may be configured to draw fluid and debris from the surgical site of a patient from the opening 80 and through the elongated probe 62 via one or more interior aspiration passages 90. In operation, debris and/or fluids in a patient cavity may be retrieved through the aspiration passage 90 and the retrieved fluids may be replaced with clear surgical fluids (e.g., saline). In various implementations, the control system 200 may coordinate the operation of the rotary cutting members 56 with the operation of the pump 88. For example, a suction rate or intensity of the pump 88 may be controlled in coordination with one or more operating sequences and corresponding duty cycles of the rotary cutting members 56. A release switch 81 may be located proximate the first end 16 for selectively releasing and/or repositioning the elongated probe 62 (FIGS. 1-4). More particularly, the release switch 81 may include a first position 81A and a second position 81B (e.g., located proximate the first end 16 and diametrically opposed with respect to the axis A). In this manner, the elongated probe 62 can be rotated between the first position 81A where the opening 80 faces the user interface 30 and the second position 81B where the opening 80 faces away from the user interface 30.

In some implementations, the control system 200 may include a control console 92 configured to communicate with the user interface 30 and/or one or more input accessories 94 (e.g., foot pedals, remote computers, interface devices, etc.) via a user communication port 95 ("Comm. Ports"). The input accessories 94 may be in communication with the control console 92 via one or more communication ports 93 that power, transmit fluids, and receive instructions from the surgical tool 10. In various implementations, the control console 92 may include a display screen 96 which may correspond to a touchscreen providing for an additional user interface associated with the surgical tool 10. Accordingly, the surgical control system 200 may be selectively configured to control the operation of the surgical tool 10 as well as various related surgical tools.

In FIGS. 7A and 7B, a disassembled view of the elongated probe 62 is shown demonstrating the rotary cutting members 56 configured to engage a hub 98 extending distally through a probe housing 100 (e.g., cylindrical housing defining the interior passage 78). In the exemplary implementation demonstrated, the surgical tool 10 may correspond to an arthroscopic shaver having the at least one cutting window 66 formed by the cutting member 56 that may be selectively rotationally aligned with the opening 80 formed through the probe housing 100 to effectuate a cutting operation with an edge 102 of the at least one cutting window 66. In this configuration, the distal end portion 60 of the elongated probe 62 may be inserted into a patient cavity to access and manipulate patient tissue by aligning the at least one cutting window 66 with a target region of a patient for treatment. As depicted, the at least one cutting window may include a pair of cutting windows 66 which may be diametrically opposed with respect to the axis A.

The rotary cutting members 56 may engage the motor 82 and surgical pump 88 via a hub adapter 104 formed in the hub 98 in connection with the probe housing 100. The interior aspiration passage 90 may provide an interior pathway for fluid and/or debris to be recovered from a surgical site as a result of suction or vacuum pressure applied within the multi-positional grip 12 or the hub 98 via an aspiration outlet 86. At the proximal end portion 64, a drive shaft 106 may be coupled to the rotary cutting members 56, which may further engage a drive assembly (e.g., the motor 82) via a shaft coupling 108. In this configuration, the rotary cutting members 56 may rotate freely within the hub 98 and the probe housing 100 about the axis A. In this way, the adjustment of a rotation of the motor 82 at the user interface 30 and/or input accessories 94 may control the rotational position θ of the rotary cutting members 56 to selectively align the cutting windows 66 to accommodate various surgical applications and cutting routines.

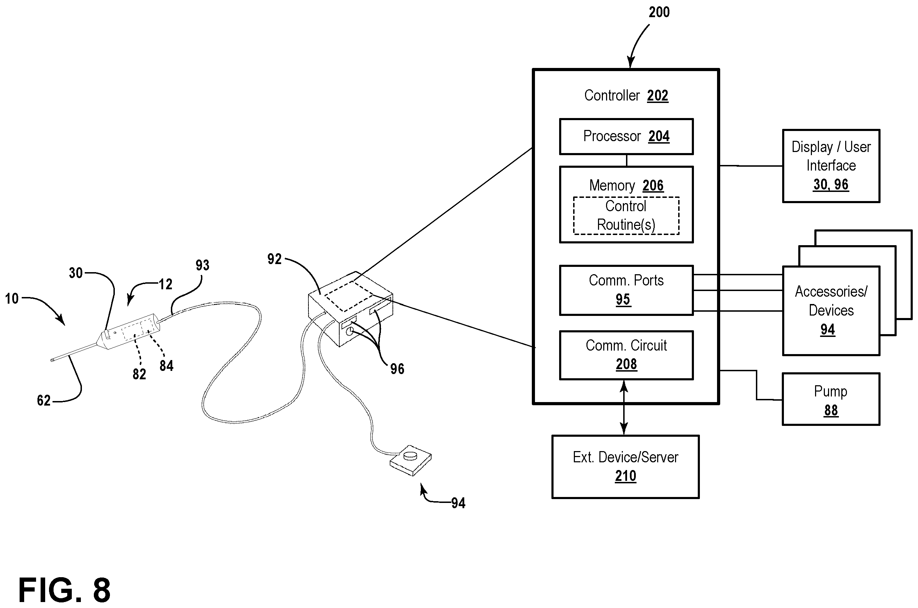

Referring now to FIG. 8, the output signals communicated from the user interface 30, the control console 92, and/or the input accessories 94 to the surgical tool 10 may be generated by various signal generators, motor controllers, or power supplies that may provide for operation of power electronic operations (e.g., motor drive signals, supply current, configuration, routine, duty cycle, etc.), which may be controlled and configured for operation based on the instructions, commands, or signals. Accordingly, the controller system 200 may be operable to generate signals to drive or control the motion, rotation, activation, intensity, and various other operating characteristics of the surgical tool 10. The control system 200 may include an electronic control unit (ECU) 202. The ECU 202 may include a processor 204 and a memory 206. The processor 204 may include any suitable processor 204 or any suitable number of processors, in addition to or other than the processor 204. In some implementations, the processor 204 may include one or more microprocessors, microcontrollers, application-specific integrated circuits (ASIC), or other circuitry configured to perform instructions, computations, and control various input/output signals to control the control system 200. The memory 206 may comprise a single disk or a plurality of disks (e.g. hard drives) that may include a storage management module that manages one or more partitions within the memory 206. In some implementations, memory 206 may include flash memory, semiconductor (solid-state) memory, or the like. The memory 206 may include Random Access Memory (RAM), a Read-Only Memory (ROM), Electrically Erasable Programmable Read-Only Memory (EEPROM), or a combination thereof. The memory 206 may include instructions that, when executed by the processor 204, cause the processor 204 to, at least, perform the functions associated with the components of the surgical tool 10.

As shown, the control system 200 may incorporate additional communication circuits or input/output circuitry, such as the communication circuit(s) 208, which may be implemented to communicate with one or more peripherals, devices, remote computers or servers 210, etc. The communication circuit(s) 208 may complement or support the operating capability of the user communication ports 95. In general, the communication circuit 208 may provide for communication via a variety of communication protocols to support operation of the surgical tool 10. In an exemplary implementation, the circuitry associated with the user communication ports 95 may include digital-to-analog converters, analog-to-digital converters, digital inputs and outputs, as well as one or more communication interfaces or buses. The circuits associated with the user communication ports 95 and/or the communication circuit 208 may be implemented with various communication protocols, such as serial communication (e.g., CAN bus, I2C, etc.), parallel communication, or network communication (e.g., RS232, RS485, Ethernet). In some cases, the communication circuit 208 may also provide for wireless network communication (Wi-Fi, Bluetooth^{®}, Ultra-wideband [UWB], etc.). In some examples, the control system 200 may be in communication with one or more of the external devices (e.g., control devices, peripherals, servers, etc.) via the communication circuit 208. Accordingly, the control console 92 may provide for communication with various devices to update, maintain, and control the operation of the surgical tool 10.

With reference now to FIG. 9, the surgical tool 10 may include a grip attachment 250. In some implementations, the grip attachment 250 may extend from the second end 18 of the multi-positional grip 12. The grip attachment 250 may include a handle 252 that allows the caregiver to steady the orientation of the probe 62 with a second hand. In some implementations, the handle 252 may define a closed loop shape. The handle 252 may be generally oriented and extend along the axis A. In some implementations, the handle 252 may extend upwardly (e.g., relative to the top surface 26 in an opposite direction from the bottom surface 20). In some implementations, the grip attachment 250 may adjustable to set a spacing between the multi-positional grip 12 and the grip attachment 250. In some implementations, the grip attachment 250 may be connected to the communication port 93 (e.g., cable, tubing, and/or the like) and further provide cable management during usage. The grip attachment 250 may include the same non-slip material and/or textures as those described above in reference to the multi-positional grip 12. In some implementations, the grip attachment 250 may be selectively coupled directly to the second end 18 of the multi-positional grip 12 and include (not shown) a surface facing away from the multi-positional grip 12 that may be selectively coupled to the communication port 93 (e.g., cable, tubing, and/or the like). In some implementations, the grip attachment 250 may be selectively coupled to the second end 18 in a variety of orientations, such that the handle 252 can be located in different positions about the axis A. The design and various features of the handle 252 may allow caregivers to grasp or engage the tool 10 based on a handedness or preference by utilizing the different holding positions P.1.-P.7.

According to some aspects of the disclosure, a multi-positional grip for a surgical tool includes a grip housing extending along an axis between a first end and a second end. A bottom surface at least partially defines a first grasping depression and a second grasping depression spaced from the first grasping depression. A top surface is spaced from the bottom surface by a pair of side surfaces. The top surface includes a user interface located proximate to the first end that includes at least one operational input.

According to various aspects, the disclosure may implement one or more of the following features or configurations in various combinations:
- the pair of side surfaces define a mean width and the top and bottom surfaces define a mean height that is greater than the mean width;
- the first grasping depression is defined by a first depression wall and a first depression floor, the first depression wall extending towards the axis to the first depression floor to define a first depth;
- the second grasping depression is defined by a second depression wall and a second depression floor, the second depression wall extending towards the axis to the second depression floor to define a second depth;
- the first depth is approximately equal to the second depth;
- the first depression wall is symmetrical along the side surfaces;
- the second depression wall is symmetrical along the side surfaces;
- the first grasping depression is located proximate the first end and the first depression wall is at least partially defined by the pair of side surfaces;
- the first depression wall includes a pair of first longitudinal portions extending between the first end and the second end, the first longitudinal portions angled from the bottom surface towards the top surface in a direction towards the second end;
- the top surface at least partially defines a third grasping depression that extends between the second end and the at least one operational input;
- the third grasping depression defines a first length along the axis and the first grasping depression defines a second a length along the axis that is less than the first length and greater than 50% of the first length;
- the first grasping depression is substantially aligned with the at least one operational input along the axis;
- the second grasping depression defines a third length that is less than the first length and greater than 50% of the first length;
- an active length of between about 100mm and about 200mm is defined between a portion of the third grasping depression nearest the second end and the at least one user input nearest the first end, and the third grasping depression extends over 50% of the active length;
- the top surface at least partially defines a third grasping depression extending between the second end and the at least one operational input that is at least partially aligned with the second grasping depression along the axis;
- the third grasping depression is at least partially aligned with the first grasping depression along the axis and the first grasping depression is at least partially aligned with the at least one operational input along the axis;
- a non-slip material covers the first grasping depression and the second grasping depression; and/or
- a rotary surgical tool includes the multi-positional grip.

According to another aspect of the disclosure, a surgical tool includes a multi-positional grip. The multi-positional grip includes a grip housing extending along an axis between a first end configured to be coupled to a surgical instrument and a second end configured to be coupled to a control console. A bottom surface at least partially defines a first grasping depression proximate the first end and a second grasping depression proximate the second end. A top surface is spaced from the bottom surface by a pair of side surfaces. The top surface defines a third grasping depression and includes a user interface that is at least partially aligned with the first grasping depression along the axis and located between the first end and the third grasping depression.

According to various aspects, the disclosure may implement one or more of the following features or configurations in various combinations:
- the user interface defines at least one operational input and an active length is defined between a portion of the third grasping depression nearest the second end and the at least one user input nearest the first end.
- the active length is between about 100mm and about 200mm.
- the first grasping depression and the second grasping depression are spaced by a bridge portion extending from the bottom surface, and the third grasping depression defines a first length extending along the axis that is greater than a length defined by a combination of the second grasping depression and the bridge portion.
- the first grasping depression is defined by a first depression wall and a first depression floor, and the first depression wall includes at least one linear portion extending substantially towards the user interface.
- the second grasping depression is defined by a second depression wall and a second depression floor, and the second depression wall includes at least one linear portion extending substantially towards the user interface.
- first grasping depression mirrors the second grasping depression along the active length.
- the third grasping depression is at least partially aligned with the first grasping depression and the second grasping depression along the axis.

According to yet another aspect of the disclosure, a multi-positional grip for a surgical tool includes a grip housing extending along an axis between a first end configured to couple to a surgical instrument and a second end. A bottom surface at least partially defines a first grasping depression proximate the first end and a second grasping depression proximate the second end and spaced from the first grasping depression. A top surface is spaced from the bottom surface by a pair of side surfaces. The top surface includes a user interface located proximate the first end and a third grasping depression proximate the second end that is at least partially axially aligned with the first grasping depression and the second grasping depression.

According to various aspects, the disclosure may implement one or more of the following features or configurations in various combinations:
- The plurality of holding positions includes two or more of a forward holding position, a backward holding position, a tripod holding position, a palmer holding position, a digital pronate holding position, a digital holding position, and a jaw chuck holding position;
- the multi-positional grip is configured to be engaged by the caregiver in a forward holding position with the multi-positional grip in a supine orientation and the surgical instrument extending away from the caregiver. The forward holding position comprises a thumb digit resting proximate the user interface, a first opposing finger located in the first grasping depression, and a second opposing finger located in the second grasping depression;
- the multi-positional grip is configured to be engaged by the caregiver in a backward holding position with the multi-positional grip in a supine orientation and the surgical instrument extending towards the caregiver. The backward holding position comprises a thumb digit resting proximate the second end, a first opposing finger located in the first grasping depression, and a second opposing finger located in the second grasping depression;
- the multi-positional grip is configured to be engaged by the caregiver in a tripod holding position with the multi-positional grip moveable between a supine and an upright orientation and the surgical instrument extending downwardly and away from the caregiver. The forward holding position comprises a thumb digit resting on a first angled surface of the user interface, a first opposing finger resting on a second angled surface of the user interface, and a portion of a palm between the thumb digit and the first opposing finger resting in the first grasping depression;
- the multi-positional grip is configured to be engaged by the caregiver in a palmer holding position with the multi-positional grip moveable between a supine and an upright orientation and the surgical instrument extending downwardly and away from the caregiver. The palmer holding position comprises a plurality of opposing fingers located in the third grasping depression, and a portion of a palm between a thumb digit and an index finger resting in the first grasping depression;
- the multi-positional grip is configured to be engaged by the caregiver in a digital pronate holding position with the multi-positional grip rotatable between a supine and a prone orientation and the surgical instrument extending away from the caregiver. The digital pronate holding position holding position comprises a first opposing finger resting on an angled side surfaces of the interface portion, a thumb digit resting on the side surface proximate the top surface, and a portion of a palm between the thumb digit and an index finger resting in the second grasping depression;
- the multi-positional grip is configured to be engaged by the caregiver in a digital holding position with the multi-positional grip in a substantially upright orientation and the surgical instrument extending downwardly. The digital holding position comprises at least three opposing fingers resting on one of the side surfaces, a thumb digit resting on the other side surface, and a palm spaced from the grip housing; and/or
- the multi-positional grip is configured to be engaged by the caregiver in a jaw chuck holding position with the multi-positional grip in a substantially upright orientation and the surgical instrument extending downwardly. The jaw chuck holding position comprises one or two opposing fingers resting on one of the side surfaces, a thumb digit resting on the other side surface, and a palm spaced from the grip housing.

It will be understood that any described processes or steps within described processes may be combined with other disclosed processes or steps to form structures within the scope of the present device. The exemplary structures and processes disclosed herein are for illustrative purposes and are not to be construed as limiting.

It is also to be understood that variations and modifications can be made on the aforementioned structures and methods without departing from the concepts of the present device, and further it is to be understood that such concepts are intended to be covered by the following claims unless these claims by their language expressly state otherwise.

As used herein, the terms "about" and "approximate" means that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art. When the term "about" is used in describing a value or an end-point of a range, the disclosure should be understood to include the specific value or end-point referred to. Whether or not a numerical value or end-point of a range in the specification recites "about," the numerical value or end-point of a range is intended to include two embodiments: one modified by "about," and one not modified by "about." It will be further understood that the end-points of each of the ranges are significant both in relation to the other end-point, and independently of the other end-point.

The terms "substantial," "substantially," and variations thereof as used herein are intended to note that a described feature is equal or approximately equal to a value or description. For example, a "substantially planar" surface is intended to denote a surface that is planar or approximately planar. Moreover, "substantially" is intended to denote that two values are equal or approximately equal. In some embodiments, "substantially" may denote values within about 10% of each other, such as within about 5% of each other, or within about 2% of each other.

The above description is considered that of the illustrated embodiments only. Modifications of the device will occur to those skilled in the art and to those who make or use the device. Therefore, it is understood that the embodiments shown in the drawings and described above are merely for illustrative purposes and not intended to limit the scope of the device, which is defined by the following claims as interpreted according to the principles of patent law, including the Doctrine of Equivalents.

## Claims

1. A multi-positional grip (12) for a surgical tool (10), the multi-positional grip (12) comprising:
a grip housing (14A, 14C, 14D) extending along an axis (A) between a first end (16) and a second end (18);
a bottom surface (20) at least partially defining a first grasping depression (22A, 22C, 22D) and a second grasping depression (24A, 24C, 24D) that is spaced from the first grasping depression (22A, 22C, 22D); and
a top surface (26) spaced from the bottom surface (20) by a pair of side surfaces (28), the top surface (26) including a user interface (30) located proximate the first end (16), the user interface (30) including at least one operational input.

2. The multi-positional grip (12) according to claim 1, wherein the pair of side surfaces (28) define a mean width and the top and bottom surface (20)s define a mean height that is greater than the mean width.

3. The multi-positional grip (12) according to any of the preceding claims , wherein the first grasping depression (22A, 22C, 22D) is defined by a first depression wall (38A, 38C, 38D) and a first depression floor (44A, 44C, 44D) (40A, 40C, 40D), the first depression wall (38A, 38C, 38D) extending towards the axis (A) to the first depression floor (44A, 44C, 44D) (40A, 40C, 40D) to define a first depth.

4. The multi-positional grip (12) according to any of the preceding claims, wherein the second grasping depression (24A, 24C, 24D) is defined by a second depression wall (42A, 42C, 42D) and a second depression floor (44A, 44C, 44D), the second depression wall (42A, 42C, 42D) extending towards the axis (A) to the second depression floor (44A, 44C, 44D) to define a second depth.

5. The multi-positional grip (12) according to claim 3 and 4, wherein the first depth is approximately equal to the second depth.

6. The multi-positional grip (12) according to claim 4 or 5, wherein the first depression wall (38A, 38C, 38D) and the second depression wall (42A, 42C, 42D) are symmetrical along the side surfaces (28).

7. The multi-positional grip (12) according to any of the preceding claims, wherein the top surface (26) at least partially defines a third grasping depression (34A, 34C, 34D) that extends between the second end (18) and the at least one operational input.

8. The multi-positional grip (12) according to claim 7, wherein the third grasping depression (34A, 34C, 34D) defines a first length along the axis (A) and the first grasping depression (22A, 22C, 22D) defines a second a length along the axis (A) that is less than the first length and greater than 50% of the first length.

9. The multi-positional grip (12) according to any of the preceding claims, wherein the first grasping depression (22A, 22C, 22D) is substantially aligned with the at least one operational input along the axis (A).

10. The multi-positional grip (12) according to claim 8 or claim 9 with reference to claim 8, wherein the second grasping depression (24A, 24C, 24D) defines a third length that is less than the first length and greater than 50% of the first length.

11. The multi-positional grip (12) according to claim 7 or claim 8, wherein an active length is defined between a portion of the third grasping depression (34A, 34C, 34D) nearest the second end (18) and the at least one user input nearest the first end (16), and the third grasping depression (34A, 34C, 34D) extends over 50% of the active length.

12. The surgical tool (10) according to claim 11, wherein the active length is between about 100mm and about 200mm.

13. The surgical tool (10) according to claim 7 or claim 8, wherein the first grasping depression (22A, 22C, 22D) and the second grasping depression (24A, 24C, 24D) are spaced by a bridge portion (54) extending from the bottom surface (20), and the third grasping depression (34A, 34C, 34D) defines a first length extending along the axis (A) that is greater than a length defined by a combination of the second grasping depression (24A, 24C, 24D) and the bridge portion (54).

14. The surgical tool (10) according to claim 7 or claim 8, wherein the third grasping depression (34A, 34C, 34D) is at least partially aligned with the first grasping depression (22A, 22C, 22D) and the second grasping depression (24A, 24C, 24D) along the axis (A).

15. The surgical tool (10) according to any of the preceding claims, wherein the first end (16) is configured to be coupled to a surgical instrument (62) and the second end (18) is configured to be coupled to a control console (92).
